(19) Europäisches Patentamt
European Patent Office
Office européen des brevets

(11) Veröffentlichungsnummer: **0 025 843**
**B1**

(12) # EUROPÄISCHE PATENTSCHRIFT

(45) Veröffentlichungstag der Patentschrift: **19.06.85**

(21) Anmeldenummer: **80104521.2**

(22) Anmeldetag: **31.07.80**

(51) Int. Cl.⁴: **C 07 D 307/86**, C 07 C 43/23, C 07 C 39/19, C 07 C 41/16, C 07 C 37/055

(54) **Herstellung von Monoalkylethern von Hydroxyphenolen und deren Umwandlung zu Hydroxycumaranen.**

(30) Priorität: **10.08.79 DE 2932458**

(43) Veröffentlichungstag der Anmeldung: **01.04.81 Patentblatt 81/13**

(45) Bekanntmachung des Hinweises auf die Patenterteilung: **19.06.85 Patentblatt 85/25**

(84) Benannte Vertragsstaaten: **BE CH DE FR GB IT LI NL**

(56) Entgegenhaltungen:
**DE - A - 1 493 691**
**DE - A - 2 845 429**
**DE - A - 2 925 763**
**US - A - 3 474 171**
**US - A - 3 923 901**

**Houben-Weyl IV / 1c, Teil 1, S.512 Fieser & Fieser,Organische Chemie, 2e Auflage, S.936-7.**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Büttner, Gerhard, Dr., Adolf-von-Menzel-Strasse 1, D-5024 Pulheim (DE)**
Erfinder: **Christmann, Karl-Friedrich, Dr., Liebermannstrasse 9, D-4047 Dormagen (DE)**
Erfinder: **Lenthe, Manfred, Dr., Michaelshöhe 40, D-5068 Odenthal (DE)**
Erfinder: **Allenbach, Udo, Dr., Stammheimer Ring 52, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 025 843 B1

BUNDESDRUCKEREI BERLIN

**Beschreibung**

Die vorliegende Erfindung betrifft ein neues Verfahren zur Monoalkylierung von Hydroxyphenolen sowie deren weitere Umwandlung zu Hydroxycumaranen.

Bei der selektiven Monoveretherung von Hydroxyphenolen können eine Reihe von Nebenreaktionen eintreten. Die unerwünschte Dietherbildung vermindert deutlich die Ausbeute an Monoether. Bei Einsatz von reaktiven Alkylierungsmitteln wie z. B. gegebenenfalls substituierten Alkylhalogeniden können in Abhängigkeit vom verwendeten Lösungsmittel darüber hinaus Kernalkylierungsprodukte gebildet werden. Will man die Dietherbildung durch nur teilweisen Umsatz des Hydroxyphenols unterdrücken, so muß bei wirtschaftlicher Fahrweise das im Überschuß vorhandene Hydroxyphenol zurückgewonnen werden. Dies ist im allgemeinen nur durch eine aufwendige und verlustreiche Extraktion mit großen Lösungsmittelmengen möglich. Eine destillative Abtrennung des gebildeten Monoethers ist im technischen Maßstab mit einem erheblichen Sicherheitsrisiko verbunden, weil sich z. B. besonders Monoalkylether von Hydroxyphenolen bei höheren Temperaturen in einer stark exothermen Reaktion in die isomeren kernsubstituierten Hydroxyphenole umlagern (Claisen-Umlagerung).

Zur Lösung der Probleme bei der Herstellung von Monoethern aus Hydroxyphenolen sind verschiedene Verfahren vorgeschlagen worden. Es ist bereits nach US-A-2 362 479 bekannt, daß sich Hydroxyphenole wie z. B. Hydrochinon in einer Suspension von $K_2CO_3$ in Ethanol mit Alkylierungsmitteln wie z. B. Methallylchlorid bei »Rückflußtemperatur« alkylieren lassen. Hydrochinon wird dabei in einem Überschuß von 100% eingesetzt.

Die Claisen-Umlagerung wird bei 225° C in Dimethylanilin als Lösungsmittel und der Ringschluß zu Hydroxycumaran mit einem Überschuß von 100% an Pyridinhydrochlorid durchgeführt. Besonders nachteilig an dem Verfahren ist in der Alkylierungsstufe der doppelt molare Einsatz an Hydrochinon, bezogen auf das Alkylierungsmittel, der durch Destillation nicht vom Monoalkylierungsprodukt abgetrennt werden kann und anschließend durch Extraktion aus einer verdünnten wäßrigen Lösung zurückgewonnen werden muß. Für die nachfolgende Claisen-Umlagerung ist ein Lösungsmittelwechsel erforderlich. Der Ringschluß zu Hydroxycumaranen wird mit der doppelt stöchiometrischen Menge an Pyridinhydrochlorid durchgeführt, obwohl solche Ringschlußreaktionen nur katalytische Mengen an Säure erfordern.

Es ist weiterhin bekannt (vgl. US-A-3 474 171), daß man Brenzkatechin mit Methallylchlorid in Gegenwart von äquivalenten Mengen an $K_2CO_3$ und KJ in Aceton als Verdünnungsmittel in das Methallyloxyphenol überführen kann, wenn man die Mischung 30 Stunden bei Rückfluß erhitzt. Die Ausbeute liegt bei 45% der Theorie. Umlagerung und Ringschluß zum 7-Hydroxycumaran werden ohne Lösungsmittel bei 200 bis 275° C durchgeführt; Ausbeuteangaben für diese Stufen fehlen. Für eine technische Durchführung besonders nachteilig sind in der Alkylierungsstufe die langen Reaktionszeiten bei geringer Ausbeute und der äquimolare Einsatz des teuren Kaliumjodid, welches für einen erneuten Einsatz nur mit großen Verlusten aus dem Salzgemisch wiedergewonnen werden kann. Eine lösungsmittelfreie Umlagerung des Methallyloxyphenols ist wegen der großen Wärmetönung nur unter hohem technischen Aufwand durchführbar.

Weiterhin ist in DE-A-2 451 936 (= US-A-3 927 118) ein Verfahren für die selektive Monoalkylierung von Hydroxyphenolen beschrieben. Dabei werden Hydroxyphenole mit Alkylierungsmitteln und Erdalkalihydroxiden oder Erdalkalioxiden als Base in dipolarer aprotischen Lösungsmitteln, die eine Sulfoxid-, Sulfon- oder Amidgruppe tragen, umgesetzt. Nachteilig an diesem Verfahren ist die Aufarbeitung. Die erhaltenen Umsetzungsprodukte (Mono- und Diether) müssen zunächst durch Extraktion aus dem Reaktionsgemisch entfernt und unter Abtrennen großer Lösungsmittelmengen isoliert werden. Danach kann das nicht umgesetzte Hydroxyphenol und das zur Alkylierung verwendete Lösungsmittel durch eine weitere Destillation vom Salzrückstand abgetrennt werden. Für eine wirtschaftliche Durchführung muß sowohl das im doppelten Überschuß eingesetzte Hydroxyphenol als auch das teure Lösungsmittel destillativ wiedergewonnen werden. Diese Rückführung ist aber mit erheblichen Verlusten verbunden.

Weiterhin ist in DE-A-2 845 429 ein Verfahren für die Herstellung von o-Methallyloxyphenol beschrieben, bei welchen in dipolar aprotischen Lösungsmitteln Brenzkatechin mit Methallylchlorid in Gegenwart von Alkalicarbonaten oder Alkalibicarbonaten umgesetzt wird, welches die Nachteile des Verfahrens nach DE-OS 2 451 936, nämlich hohe Brenzkatechinüberschüsse, auszugleichen sucht. Es gelingt aber auch hier nicht, bei einem guten Mono/Diether-Verhältnis Brenzkatechin vollständig umzusetzen. Andererseits ist die destillative Abtrennung des Monoethers von den gewählten dipolar aprotischen Lösungsmitteln wegen der thermischen Instabilität des Monoethers und den hohen Siedepunkten der Lösungsmittel problematisch und schwierig. Eine Extraktion ist mit den bereits in DE-OS 2 451 936 beschriebenen Nachteile verbunden.

Es wurde gefunden:

1. Ein Verfahren zur Herstellung von 7-Hydroxycumaranen der Formel (I)

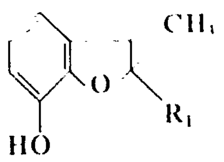

(I)

in welcher

$R^1$ für Wasserstoff oder $C_{1-4}$-Alkyl steht,

durch Umsetzung von Brenzkatechin der Formel (II)

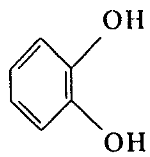

(II)

mit Allylverbindungen der Formel (III)

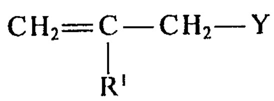

(III)

in welcher

$R^1$ für Wasserstoff oder $C_{1-4}$-Alkyl steht und
Y für Halogen, niederes Alkylsulfonat oder Arylsulfonat steht,

indem in erster Stufe eine Monoveretherung durchgeführt wird, und indem in zweiter Stufe eine Umlagerung des in der ersten Stufe gebildeten Brenzkatechinmonoethers durchgeführt wird, und indem in dritter Stufe eine Cyclisierung des in der zweiten Stufe gebildeten 3-Alkenylbrenzkatechins durchgeführt wird, dadurch gekennzeichnet, daß in allen Stufen als Lösungsmittel ein Glycolmonoalkylether mit 1—4 C-Atomen in der Alkylgruppe des Alkoxyrestes verwendet wird, daß in der zweiten Stufe bei pH 2—8 gearbeitet wird, daß in der dritten Stufe bei kurzen Verweilzeiten von 1—30 Minuten und engem Verweilzeitspektrum mit einer Bodensteinzahl >5 gegebenenfalls in Anwesenheit von Eisensalzen sowie in Anwesenheit saurer Katalysatoren in Konzentrationen von 0,2—2,5 Gewichtsprozent gearbeitet wird.

Vorzugsweise wird in der dritten Stufe in Anwesenheit von Eisensalzen gearbeitet.

Bevorzugte Substituenten Y in Formel (III) sind Halogen, insbesondere Chlor, $C_{1-4}$-Alkylsulfonyl, Phenyl- oder p-Tolylsulfonyl.

Es ist als ausgesprochen überraschend zu bezeichnen, daß durch Wahl von Glycolmonoalkylethern mit 1—4 C-Atomen in der Alkylgruppe des Alkoxyrestes als Verdünnungsmittel in der 1. Stufe die Hydroxyphenolether in hohen Ausbeuten bei kurzen Reaktionszeiten erhalten werden. Die hohen Ausbeuten werden ohne Zusatz eines Cokatalysators erzielt. Trotz Überschuß an Alkylierungsmittel ist die Bildung des entsprechenden Diethers gering. Ein hoher bis quantitativer Hydroxyphenolumsatz ist somit möglich bei hoher Selektivität der Monoetherbildung und gleichzeitiger Auskreisung des Reaktionswassers.

Es war ferner überraschend, daß die 2. Stufe ohne Schwierigkeiten und mit guten Ausbeuten abläuft, wenn man in einem pH-Bereich von 2—8 sowie im selben Lösungsmittel wie in der 1. Stufe arbeitet.

Es war ferner überraschend, daß auch in der 3. Stufe mit guter Ausbeute gerechnet werden kann, wenn man bei kurzen Verweilzeiten von 1—30 Minuten, bevorzugt 5—15 Minuten, und engem Verweilzeitspektrum mit einer Bodenstein-Zahl >5 sowie bevorzugt in Anwesenheit von Eisensalzen als Cokatalysatoren sowie in Gegenwart saurer Katalysatoren arbeitet. (Zur Definition der Bodensteinzahl vgl. E. Fitzer, W. Fritz, Technische Chemie, Springer Verlag Berlin, 1975, S. 346.)

Verwendet man Brenzkatechin und Methallylchlorid als Ausgangsstoffe, so kann der Reaktionsverlauf bei Stufe 1 durch folgendes Formelschema wiedergegeben werden:

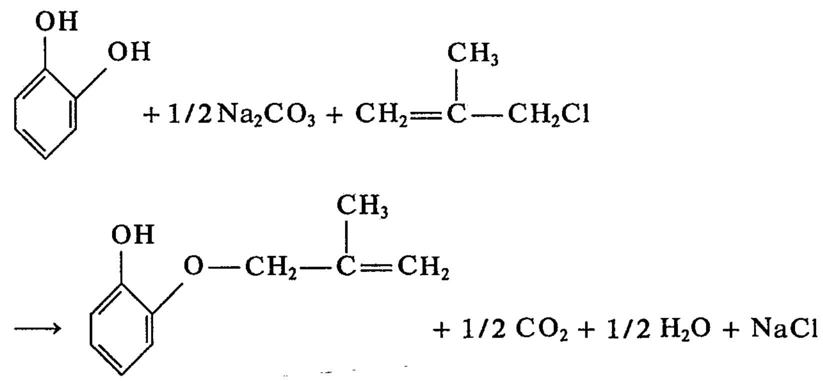

Bei der Durchführung der 1. Stufe des erfindungsgemäßen Verfahrens läßt man 1 Mol Hydroxyphenol mit 1,1—2,5 Mol Alkylierungsmittel reagieren. Bevorzugt ist das Verhältnis 1,25—1,8 Mol Alkylierungsmittel pro Mol Hydroxyphenol. Das Verdünnungsmittel wird im Verhältnis 1,5—5,0 Gewichtsteilen pro Gewichtsteil Hydroxyphenol eingesetzt. Es wird in Anwesenheit einer Base gearbeitet. Als solche seien genannt Alkali- oder Erdalkalihydroxide, -carbonate oder -hydrogencarbonate. Bevorzugt sind Natriumcarbonat oder Natriumhydrogencarbonat. Es kann auch in Anwesenheit basischer Ionenaustauscher anstelle von Basen gearbeitet werden.

Günstig ist das Verhältnis 2,0—4,0 kg Verdünnungsmittel pro kg Hydroxyphenol. Der Zusatz der Alkalibase erfolgt im Verhältnis 1,0—2,0 Basenäquivalente pro Mol Hydroxyphenol. Es empfiehlt sich unter leichtem Stickstoffüberdruck sowie unter Zugabe eines Reduktionsmittels wie Natriumdithionit zu arbeiten. Es wird bei Temperaturen zwischen 60 und 140°C, bevorzugt zwischen 85 und 125°C, gearbeitet.

Unter den als Verdünnungsmittel verwendeten Glykolmonoalkylethern mit 1—4 C-Atomen in der Alkylgruppe des Alkoxyrestes ist der Glykolmonomethylether in allen drei Verfahrensstufen besonders bevorzugt.

Das in der Reaktion gebildete oder durch die Einsatzstoffe eingeschleppte Wasser wird während der Reaktion oder im Anschluß daran durch Destillation eines Azeotrops entfernt.

Die bei der Reaktion gebildeten Salze können im Anschluß an die Reaktion durch Filtration bequem abgetrennt werden.

Durch die Möglichkeit, das Reaktionswasser erst nach Ablauf der Reaktion aus dem Produktgemisch zu entfernen, bietet sich vor allem bei größeren Produktionsraten die kontinuierliche Durchführung der Monoetherbildung an. Die Umsetzung kann dann z. B. in einer Rührkesselkaskade erfolgen, in deren erste Stufe alle Reaktionspartner eingegeben werden. Das Reaktionswasser wird nach der letzten Stufe destillativ und die Salze werden durch Filtration entfernt.

Verwendet man Brenzkatechinmonomethallylether als Ausgangsstoff, so läßt sich der Reaktionsverlauf der 2. und 3. Stufe durch folgendes Formelschema wiedergeben:

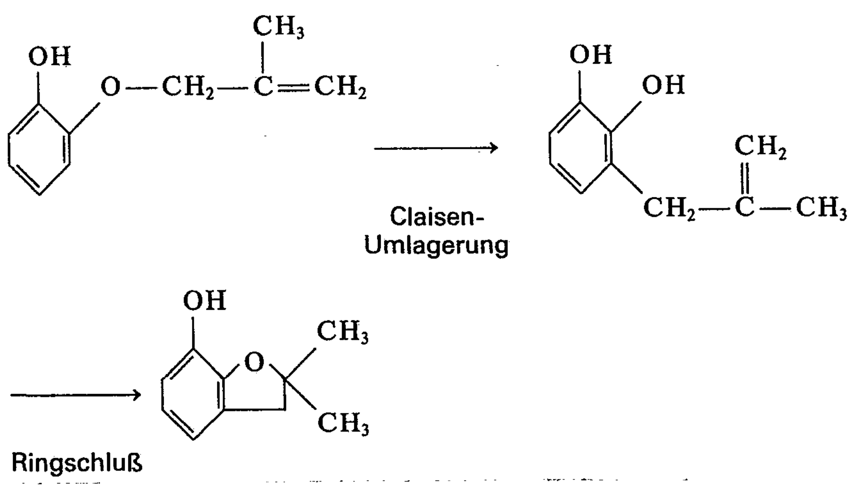


Für die Durchführung der 2. Stufe des erfindungsgemäßen Verfahrens wird nach Abdestillieren des Azeotrops das im Überschuß eingesetzte Alkylierungsmittel abdestilliert. Dieses kann direkt wieder in die 1. Stufe zur Monoetherherstellung verwendet werden.

Anschließend trennt man den ausgefallenen Salzniederschlag ab und setzt die so erhaltene Lösung des Monoethers in die Claisen-Umlagerung ein. Die Filtration kann aber auch nach der Wasserauskreisung vorgenommen werden.

Die Umlagerung kann kontinuierlich oder diskontinuierlich bei Temperaturen von 140—220°C unter Druck oder auch bei Normaldruck durchgeführt werden. Bevorzugt ist ein Temperaturbereich von 150—190°C. Wegen der relativ hohen Wärmetönung der Claisen-Umlagerung ist eine kontinuierliche Verfahrensführung zweckmäßiger.

In der 2. Stufe arbeitet man bevorzugt in einem pH-Bereich von 2—8. Der pH-Wert oder entsprechend der Säure- oder Basengehalt der Lösung des Monoethers in dem Glycolmonoalkylether mit 1—4 C-Atomen in der Alkylgruppe des Alkoxyrestes läßt sich durch eine Glaselektrode bzw. durch eine potentiometrische Titration mittels 1/10 n-NaOH oder 1/10 n-HCl nach Verdünnen mit Wasser bestimmen. Die Durchführung der 2. Stufe schließt sich ohne Wechsel des Verdünnungsmittels an die 1. Stufe an.

Für die Durchführung der 3. Stufe des erfindungsgemäßen Verfahrens wird die Lösung des 3-Alkenylbrenzkatechins aus der Stufe 2 mit einem sauren Katalysator (gelöst im verwendeten Lösungsmittel) versetzt und auf Temperaturen von 120—230°C, bevorzugt zwischen 140—220°C erhitzt. Der Ringschluß zu 7-Hydroxycumaranen kann kontinuierlich oder diskontinuierlich durchgeführt werden.

Wesentlich für die erfindungsgemäße Durchführung der 3. Stufe ist das Arbeiten bei kurzen Verweilzeiten von 1—30 Minuten und engem Verweilzeitspektrum mit einer Bodensteinzahl von >5. Die Anwesenheit von Eisensalzen als Katalysatoren ist besonders bevorzugt.

Bei dem sauren Katalysator der Stufe III kann es sich um Mineralsäuren wie z. B. HCl, $H_2SO_4$, $H_3PO_4$ oder $H_3BO_3$ oder um starke organische Säuren wie z. B. Sulfonsäuren oder Carbonsäuren handeln. Besonders bevorzugt ist p-Toluolsulfonsäure, die im verwendeten Lösungsmittel flüssig dosiert werden kann. Eisensalze wie $FeCl_3$ zeigen eine zusätzliche katalytische Wirksamkeit. Die Konzentration des Katalysators liegt bei 0,2%—2,5 Gew.-% (bez. auf die eingesetzte Lösung), bevorzugt bei 0,8%—1,7 Gew.-%.

Die Stufen 1—3 werden bevorzugt unter Druck kontinuierlich oder diskontinuierlich durchgeführt (Stufe 1 kann sowohl bei Normal- als auch unter erhöhtem Druck geführt werden).

Die Isolierung des gebildeten 7-Hydroxycumarans erfolgt direkt durch Destillation der organischen Phase aus der Cyclisierungsreaktion. Zusätzliche Reinigungsschritte sind nicht erforderlich. Das dabei in hohen Ausbeuten erhaltene Lösungsmittel kann ebenfalls direkt wieder in die 1. Stufe eingesetzt werden.

Durch die Auskreisung des Reaktionswassers trennen sich die während der Alkylierungsreaktion entstehenden Salze quantitativ ab und können problemlos durch Filtration entfernt werden. Für die Folgereaktion zu Hydroxycumaranen ist kein Lösungsmittelwechsel erforderlich. Es kann direkt das Filtrat aus der Monoetherstufe eingesetzt werden. Bei druckloser Fahrweise der Stufen 2 und 3 wird ein Großteil des verwendeten Lösungsmittels- und der Alkylierungsmittelüberschuß während der Umlagerung und Cyclisierung entfernt.

Beim Arbeiten unter Druck wird vor Durchführung der Stufen 2 und 3 Alkylierungsmittel durch Destillation abgetrennt. Die Hauptmenge des Lösungsmittels verbleibt während der Claisen-Umlagerung und der Cyclisierung im Reaktionsgemisch und wird erst bei der Aufarbeitung des Hydroxycumarans wiedergewonnen.

Die wiedergewonnenen Lösungs- und Alkylierungsmittel werden der Alkylierungsstufe nach Aufarbeitung wieder zugeführt.

Die Produkte Brenzkatechinmonomethallylether und 2,2-Dimethyl-7-hydroxycumaran sind bekannt und finden zur Herstellung von insektiziden (pestiziden) Pflanzenschutzmitteln Verwendung.

## Beispiel 1

In einem 2,5-l-Glasreaktor mit aufgesetzter kleiner Kolonne und einem Wasserabscheider werden 1150 g Glykolmonomethylether, 337 g Brenzkatechin technisch (3 Mol), 178 g (1,68 Mol) wasserfreie Soda und 5 g Natriumdithionit vorgelegt. Man leitet einen schwachen $N_2$-Strom durch die gut gerührte Suspension und heizt auf ca. 110°C Innentemperatur auf. Über einen Tropftrichter oder eine Dosierpumpe werden im Verlauf von 1 Stunde 407 g (4,5 Mol) Methallylchlorid (MAC) (bei Kreislauffahrweise eine entsprechende Menge an Rückführ-MAC) gleichmäßig zudosiert. Bereits beim Aufheizen und während der nachfolgenden Reaktion setzt ein lebhafter $CO_2$-Strom ein. Während der gesamten Reaktionsdauer von 4,5 Stunden (einschließlich Dosierung) wird das gebildete Reaktionswasser weitgehend ausgeschleust. Man kühlt auf 20°C ab und filtriert das gebildete Kochsalz ab. Den farblosen Filterrückstand wäscht man zweimal mit wenig Lösungsmittel nach und vereinigt Filtrat- und Waschlösungsmittelmengen. Die so erhaltene organische Phase wird direkt für die nachfolgende Claisen-Umlagerung und Ringschluß zu 7-Hydroxycumaran eingesetzt. Nach Gaschromatogramm enthält die organische Phase 382 g Brenzkatechinmonomethallylether (Kp=58°C bei 0,1 Torr), das sind 78% Ausbeute der Theorie. An Diether (Kp=83°C bei 0,1 Torr) sind 52 g entstanden, das sind 8% der Theorie. Das nicht umgesetzte Brenzkatechin stört die weiteren Umsetzungen nicht. Eine destillative Auftrennung der organischen Phase in die Einzelkomponenten ist wegen der thermischen Empfindlichkeit des Mono- und Diethers mit großen Verlusten und der Gefahr einer unkontrollierten exothermen Umlagerung verbunden. Eine Abtrennung des Lösungsmittels und des Methallylchloridüberschusses im Wasserstrahlvakuum ist möglich.

Beispiel 2

In einem Glasreaktor werden 337 g Brenzkatechin technisch (3 Mol), 197 g (1,85 Mol) wasserfreie Soda, 3 g Natriumdithionit und 1150 g Glykolmonomethylether (bei Kreisläufen eine entsprechende Menge aus der Aufarbeitung) vorgelegt.

Man heizt unter einem schwachen $N_2$-Strom die Suspension auf 110° C auf und dosiert im Verlauf von 45 Minuten 443 g (4,90 Mol) Methallylchlorid zu (Ausschleusung des Reaktionswassers). Sobald die $CO_2$-Entwicklung nachläßt (ca. 4,5—5 Stunden) wird auf 20° C abgekühlt, vom Niederschlag abgesaugt und dieser 2mal mit wenig Lösungsmittel nachgewaschen. Nach Gaschromatogramm sind in der so erhaltenen organischen Phase 399 g Brenzkatechinmonomethylallylether (81% der Theorie) und ca. 89 g Diether (13,6%) enthalten. Brenzkatechin ist praktisch nicht mehr nachweisbar. Die Weiter- oder Aufarbeitung erfolgt analog Beispiel 1.

Beispiel 3

In einem Glasreaktor gemäß Beispiel 1 werden 337 g Brenzkatechin technisch (3 Mol), 178 g (1,68 Mol) wasserfreie Soda und 5 g Natriumdithionit in 950 g Glykolmonomethylether vorgelegt. Man heizt auf 110° C unter Rühren auf und dosiert im Verlauf von 1—1,5 Stunden 543 g Methallylchlorid technisch (6 Mol) ein. Es setzt sofort eine kräftige $CO_2$-Entwicklung ein. Nach 4,5 Stunden Gesamtreaktionsdauer (Wasserausschleusung) lassen $CO_2$-Entwicklung und Wasserabscheidung deutlich nach. Nach dem Abkühlen wird der Salzniederschlag abfiltiert und nachgewaschen. Die organische Phase enthält nach Gaschromatogramm 384 g Brenzkatechin-monomethallylether (78% der Theorie) 8 g 3-Methallylbrenzkatechin (1,6%) und 78 g Diether (12% der Theorie). Nicht umgesetztes Brenzkatechin sowie der gebildete Diether bzw. seine Umlagerungsprodukte werden nach Claisen-Umlagerung und Ringschluß vom gebildeten 7-Hydroxycumaran destillativ abgetrennt.

Beispiel 4

In einem Glasreaktor gemäß Beispiel 1 werden 330 g Brenzkatechin (3 Mol), 260 g Natriumhydrogencarbonat (3,1 Mol), 3 g Natriumdithionit und 1150 g Methylglykol vorgelegt, unter einem schwachen $N_2$-Strom auf 95—105° C aufgeheizt und in 2 Stunden gleichmäßig 407 g (4,5 Mol) Methallylchlorid zugetropft.

Nach 4,5 Stunden Gesamtreaktionsdauer ($H_2O$-Ausschleusung) bei 105° C wird abgekühlt und die dunkelgelbe Suspension abgesaugt. Der farblose Salzniederschlag wird abgesaugt und nachgewaschen. Die organische Phase enthält nach GC 365 g Brenzkatechinmonomethallylether (74%) und 63 g Diether (9,5%). Lösungsmittel und nicht umgesetztes Methallylchlorid können nach Aufarbeitung im nächsten Ansatz wieder eingesetzt werden.

Beispiel 5

In einem Glasreaktor analog Beispiel 1 legt man 330 g Brenzkatechin (3 Mol), 350 g Soda (3 Mol) und 5 g Natriumdithionit in 1200 g n-Butanol vor und dosiert unter Rühren bei 105—110° C 407 g (4,5 Mol) Methallylchlorid in 1 Stunde zu. Man läßt noch ca. 7 Stunden bei 110° C weiterrühren und trennt das ausgeschleuste Wasser jeweils ab. Nach dem Abkühlen wird abgesaugt und mit wenig n-Butanol nachgewaschen. Die organische Phase enthält nach GC 238 g Brenzkatechinmonomethallylether (48,5% der Theorie) sowie 18 g Diether (2,8% der Theorie).

Beispiel 6

In einem 270-l-Email-Kessel mit aufgesetzter Kolonne und einem Wasserabscheider werden 33,0 kg (300 Mol) Brenzkatechin (technisch, 20,7 kg (195 Mol) wasserfreie Soda und 0,33 kg Natriumdithionit vorgelegt, mit Stickstoff gespült und nach Versetzen mit 113,6 kg Glykolmonomethylether unter Rühren suspendiert. Nach Erhitzen der gut gerührten Suspension auf 110° C Innentemperatur werden 43,5 kg (480 Mol) Methallylchlorid innerhalb von 2 Stunden bei 110° C mit einer Dosierpumpe zugepumpt. Während der Zugabe des Methallylchlorids beginnt die Suspension zu sieden und gebildetes Reaktionswasser wird mit dem über einen Wasserabscheider geführten Destillat während der gesamten Reaktionszeit von 4,5 Stunden (einschließlich Dosierung) ausgekreist.

Nach Ablauf der Reaktionszeit wird das nicht umgesetzte Methallylchlorid als Azeotrop mit nicht ausgekreistem Reaktionswasser ohne Rückführung bei einem Druck von 500 mbar abdestilliert. Danach werden bei einem Druck von 200 mbar ca. 40 kg Lösungsmittel abdestilliert, wobei noch restliches Reaktionswasser praktisch vollständig entfernt wird.

Die Suspension wird nachfolgend auf 20°C abgekühlt und über eine Nutsche filtriert, um den Feststoff abzutrennen. Der Filterkuchen wird zweimal mit je 10 kg Glykolmonomethylether gewaschen und das Waschfiltrat mit dem Filtrat vereint. Die erhaltene Lösung enthält nach Gaschromatogramm 37,5 kg Brenzkatechin-monomethallyl-ether (76% der Theorie) und 8,5 kg Brenzkatechindimethallylether (13% der Theorie).

Beispiel 7

In einem 270-l-Emailkessel werden 33,0 kg (300 Mol) Brenzkatechin (technisch), 20,7 kg (195 Mol) wasserfreie Soda und 0,33 kg Natriumdithionit vorgelegt, mit Stickstoff gespült und nach Zugabe von 113,6 kg Glykolmonomethylether unter Rühren suspendiert. Bei gutem Rühren wird die Suspension auf 110°C erhitzt. Dabei wird bereits Kohlendioxid frei und Kesselinnendruck von ca. 2,8 bar (absolut) baut sich auf. Nachfolgend werden 43,5 kg (480 Mol) Methallylchlorid innerhalb von 2 Stunden bei 110°C Innentemperatur mit einer Dosierpumpe zugepumpt, wobei der Kesselinnendruck durch teilweises Entspannen auf 6 bar (absolut) gehalten wird.

Nach einer Gesamtreaktionszeit von 4,5 Stunden (einschließlich Dosierung) wird auf 20°C abgekühlt und der Druck entspannt. Das nicht umgesetzte Methallylchlorid wird im Azeotrop mit Reaktionswasser bei 500 mbar abdestilliert.

Danach werden 40 kg Lösungsmittel bei 100 mbar abdestilliert, die das restliche Reaktionswasser enthalten. Es wird auf 20°C abgekühlt und der Feststoff durch Filtrieren über eine Nutsche abgetrennt.

Der Filterkuchen wird zweimal mit je 10 kg Glykolmonomethylether gewaschen und Filtrat und Waschfiltrate werden vereint. Die erhaltene Lösung enthält nach Gaschromatogramm 37,9 kg Brenzkatechin-monomethallylether (77% der Theorie) und 6,8 kg Brenzkatechindimethallylether (10% der Theorie).

Beispiel 8

In einem 270-l-Kessel mit aufgesetzter Kolonne werden 33,0 kg (300 Mol) Brenzkatechin (technisch), 20,7 kg (195 Mol) wasserfreie Soda und 0,33 kg Natriumdithionit vorgelegt, mit Stickstoff gespült und nach Zugabe von 113,6 kg Glykolmonomethylether unter Rühren suspendiert. Nach Erhitzen der gut gerührten Suspension auf 110°C Innentemperatur werden 43,5 kg (480 Mol) Methallylchlorid innerhalb von 2 Stunden bei 110°C mit einer Dosierpumpe zugepumpt. Die Suspension beginnt dabei zu sieden und wird über die Kolonne unter Rückfluß gehalten. Nach Ablauf der Reaktionszeit von 4,5 Stunden (einschließlich Dosierung) wird das Kondensat abgenommen, um überschüssiges Methallylchlorid im Azeotrop mit Wasser abzudestillieren. Die weitere Aufarbeitung erfolgt wie unter Beispiel 7 beschrieben. Nach Gaschromatogramm enthält die erhaltene Lösung 38,55 kg Brenzkatechin-monomethallylether (78% der Theorie) und 7,4 kg Brenzkatechin-dimethallylether (11% der Theorie).

Beispiel 9

Das Methallylchlorid in der Brenzkatechinmonomethallyletherlösung (ca. 5%) wird bei 65° im Vakuum destillativ über eine Kolonne entfernt. Die so erhaltene Lösung enthält 17,6% Brenzkatechinmonomethallylether und 0,2% 3-Methallylbrenzkatechin, das sind 17,8% an verwertbaren Produkten. Die Bezeichnung: »Verwertbare Produkte« ist die Summe von Brenzkatechinmonomethallylether als Hauptbestandteil und 3-Methallylbrenzkatechin, 3-Isobutenylbrenzkatechin und eventuell 7-Hydroxycumaran als Nebenbestandteile.

640 g dieser Lösung werden stündlich in die in Beispiel 9 beschriebene 3stufige Kesselkaskade über eine Pumpe eingeführt. Der 1. Reaktor wird bei einer Verweilzeit von 1,25 Stunden und bei 170°C und der 2. Reaktor bei einer Verweilzeit von 0,75 Stunden und bei 180°C betrieben, wobei Methylglykol jeweils über Kolonne I und II abdestilliert.

In den 3. Reaktor, der eine Verweilzeit von 0,5 Stunden und eine Temperatur von 180°C aufweist, werden noch 0,75% in Methylglykol gelöste p-Toluolsulfonsäure, bezogen auf die stündliche Durchlaufmenge, eindosiert. Zusätzlich wird eine geringe Menge Destillat der 1. Kolonne eingeführt. Die den Reaktor 3 verlassende Reaktionslösung enthält laut gaschromatographischer Analyse 74 g bis 80 g 7-Hydroxycumaran pro Stunde. Das sind 65—70% der Ausbeute der Theorie, bezogen auf die verwertbaren Produkte in der Ausgangslösung. Die Aufarbeitung erfolgt wie in Beispiel 9 beschrieben.

Beispiel 10

(Claisen-Umlagerung in 3stufiger Rührkesselkaskade)

In eine Kaskade aus zwei Email-Kesseln mit je 50 l Volumen werden bei 180° C und 6 bar stündlich 20 l einer Lösung von 15% Methallyloxyphenol in Glykolmonomethylether eingegeben. Das abfließende Reaktionsprodukt enthielt nach gaschromatographischer Analyse 12,1% verwertbarer Produkte (Ausbeute: 80,7%).

Beispiel 11

Die im Beispiel 10 hergestellte Lösung wurde nach Zusatz von 1,5% p-Toluolsulfonsäure in ein beheiztes Strömungsrohr von 6 mm Innendurchmesser und einem Volumen von 490 $cm^3$ eingespeist. Bei einer Heizmitteltemperatur von 180° C und einer Verweilzeit von 7 Minuten wurde ein Produktstrom erhalten, der nach Beispiel 9 aufgearbeitet wird. Die Ausbeute an 7-Hydroxycumaran, bezogen auf die in diesem Beispiel eingesetzte Lösung, betrug 91%.

Beispiel 12

In der Einsatzlösung des Beispiels 11 wurden 1% Eisen(III)-chlorid gegeben und dieses Gemisch nach den Bedingungen des Beispiels 14 zu 7-Hydroxycumaran umgesetzt. Die Ausbeute betrug in diesem Fall 97%, bezogen auf die in das Strömungsrohr eingesetzte Lösung. Damit wird eine Gesamtausbeute von 78% erreicht, bezogen auf eingesetztes Methallyloxyphenol.

**Patentanspruch**

Verfahren zur Herstellung von 7-Hydroxycumaranen der Formel (I)

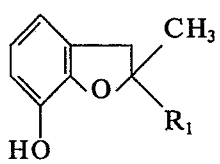

(I)

in welcher

$R^1$ für Wasserstoff oder $C_{1-4}$-Alkyl steht,

durch Umsetzung von Brenzkatechin der Formel (II)

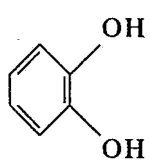

(II)

mit Allylverbindungen der Formel (III)

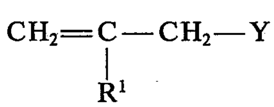

(III)

in welcher

$R^1$ für Wasserstoff oder $C_{1-4}$-Alkyl steht und
Y für Halogen, niederes Alkylsulfonat oder Arylsulfonat steht,

indem in erster Stufe eine Monoveretherung durchgeführt wird, und indem in zweiter Stufe eine Umlagerung des in der ersten Stufe gebildeten Brenzkatechinmonoethers durchgeführt wird, und indem in dritter Stufe eine Cyclisierung des in der zweiten Stufe gebildeten 3-Alkenylbrenzkatechins durchgeführt wird, dadurch gekennzeichnet, daß in allen Stufen als Lösungsmittel ein Glycolmonoalkylether mit 1—4 C-Atomen in der Alkylgruppe des Alkoxyrestes verwendet wird, daß in der zweiten

Stufe bei pH 2—8 gearbeitet wird, daß in der dritten Stufe bei kurzen Verweilzeiten von 1—30 Minuten und engem Verweilzeitspektrum mit einer Bodensteinzahl >5 gegebenenfalls in Anwesenheit von Eisensalzen sowie in Anwesenheit saurer Katalysatoren in Konzentrationen von 0,2—2,5 Gewichtsprozent gearbeitet wird.

**Claim**

Process for the preparation of 7-hydroxycoumarans of the formula (I)

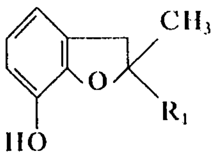

(I)

in which

$R^1$ represents hydrogen or $C_1-_4$ alkyl,

by reacting pyrocatechol of the formula (II)

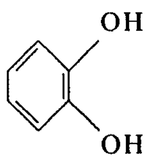

(II)

with allyl compounds of the formula (III)

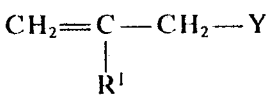

(III)

in which

$R^1$ represents hydrogen or $C_1-_4$-alkyl and
Y represents halogen, or lower alkyl sulphonate or aryl sulphonate,

by carrying out mono-etherification in a first stage and carrying out, in a second stage, rearrangement of the pyrocatechol monoether formed in the first stage, and by carrying out, in a third stage, cyclisation of the 3-alkenylpyrocatechol formed in the second stage, characterised in that in all stages a glycol monoalkyl ether with 1—4 C atoms in the alkyl group of the alkoxy radical is used as the solvent, in that the second stage is carried out at pH 2—8, in that the third stage is carried out with short residence times of 1—30 minutes and a narrow residence time spectrum with a Bodenstein index of >5, optionally in the presence of iron salts and in the presence of acid catalysts in concentrations of 0.2—2.5 percent by weight.

**Revendication**

Procédé de préparation de 7-hydroxycoumaranes de formule (I)

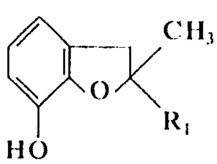

(I)

dans laquelle

$R^1$ représente un atome d'hydrogène ou un groupe alkyle en $C_1$ à $C_4$,

par réaction de la pyrocatéchine de formule (II)

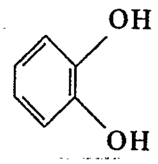

(II)

avec des composés allyliques de formule (III)

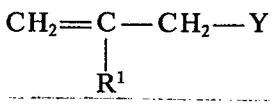

(III)

dans laquelle

$R^1$ représente un atome d'hydrogène ou un groupe alkyle en $C_1$ à $C_4$, et
Y représente un atome d'halogène, un groupe alkylsulfonate inférieur ou arylsulfonate,

selon lequel, dans une première étape, on effectue une monoétherification et, dans une seconde étape, une transposition du monoéther de pyrocatéchine formé à la première étape, et, dans une troisième étape, une cyclisation de la 3-alcénylpyrocatéchine formée dans la seconde étape, procédé caractérisé en ce que l'on utilise dans l'ensemble des trois étapes, comme solvant, un éther monoalkylique de glycol comportant 1 à 4 atomes de carbone dans le groupe alkyle du radical alcoxy; on travaille dans la seconde étape un pH de 2 à 8; on travaille dans la troisième étape avec de courts temps de séjour de 1 à 30 min et un spectre étroit du temps de séjour avec un indice de fond de creuset > à 5, éventuellement en présence de sels de fer ainsi qu'en présence de catalyseurs acides, présents en des concentration de 0,2 à 2,5% en poids.